(19) Europäisches Patentamt

European Patent Office

Office européen des brevets

(11) Veröffentlichungsnummer: **0 248 422**
**B1**

(12) # EUROPÄISCHE PATENTSCHRIFT

(45) Veröffentlichungstag der Patentschrift:
19.04.89

(51) Int. Cl.⁴: **C07C 7/06**, C07C 13/20

(21) Anmeldenummer: 87108065.1

(22) Anmeldetag: 04.06.87

(54) Verfahren zur destillativen Aufarbeitung von Cyclohexen, Cyclohexan und Benzol enthaltenden Gemischen.

(30) Priorität: 06.06.86 DE 3619173

(43) Veröffentlichungstag der Anmeldung:
09.12.87 Patentblatt 87/50

(45) Bekanntmachung des Hinweises auf die Patenterteilung:
19.04.89 Patentblatt 89/16

(84) Benannte Vertragsstaaten:
BE CH DE ES FR GB IT LI NL

(56) Entgegenhaltungen:
EP-A- 0 055 495
DE-C- 855 547
FR-A- 1 427 065
GB-A- 966 923
US-A- 2 890 154

CHEMICAL ABSTRACTS, Band 100, Nr. 9, 27.
Februar 1984, Columbus, Ohio, USA. ASAHI CHEMICAL
INDUSTRY: "Separation of moncyclic cycloalkanes",
Seite 565, Spalte 2, Zusammenfassung-Nr. 67896y & JP
A 58 164 525
CHEMICAL ABSTRACTS, Band 100,
Nr. 9, 27 Februar 1984, Columbus, Ohio, USA. ASAHI
CHEMICAL INDUSTRY: "Separation of monocyclic
cycloalkanes", Seite 565, Spalte 2, Zusammenfassung
Nr. 67897z & JP A 58 164 524

(73) Patentinhaber: BASF Aktiengesellschaft,
Carl-Bosch-Strasse 38, D-6700 Ludwigshafen(DE)

(72) Erfinder: Rieber, Norbert, Dr., Liebfrauenstrasse 1 c,
D-6800 Mannheim 51(DE)
Erfinder: Fischer, Rolf, Dr., Bergstrasse 98,
D-6900 Heidelberg(DE)
Erfinder: Brunner, Erwin, Dr., Neuhausener Weg 1,
D-6700 Ludwigshafen(DE)
Erfinder: Luyken, Herman, Bruesseler Ring 34,
D-6700 Ludwigshafen(DE)
Erfinder: Vagt, Uwe, Dr., Paul-Neumann-Strasse 44,
D-6720 Speyer(DE)

(56) Entgegenhaltungen: (Fortsetzung)

PATENT ABSTRACTS OF JAPAN, unexamined
applications, Sektion C, Band 2, Nr. 41, 17. März 1978.
THE PATET OFFICE JAPANESE GOVERNMENT,
Seite 4756 C 77

## Beschreibung

Die Erfindung betrifft ein Verfahren zur destillativen Aufarbeitung von Cyclohexen, Cyclohexan und Benzol enthaltenden Gemischen.

Derartige Gemische fallen z.B. bei der partiellen Hydrierung von Benzol und der partiellen Dehydrierung von Cyclohexan zu Cyclohexen an, wobei man neben dem gewünschten Verfahrensprodukt Cyclohexen stets auch noch Cyclohexan bzw. Benzol erhält.

Cyclohexen ist ein wichtiges Zwischenprodukt und wird großtechnisch durch Dehydratisierung von Cyclohexanol oder Dehydrohalogenierung von Chlorcyclohexan hergestellt. Kostengünstiger wäre das Hydrier- bzw. Dehydrierverfahren, wobei aber die Isolierung des Cyclohexens aus seinen Gemischen mit Benzol und Cyclohexan wegen der eng beieinanderliegenden Siedepunkte dieser drei Hauptkomponenten erhebliche Schwierigkeiten bereitet.

Die Normalsiedepunkte von Cyclohexen, Cyclohexan und Benzol liegen bei 82,1, 80,7 und 80,1°C. Azeotrope bilden Cyclohexen und Benzol bei 78,9 und Cyclohexan und Benzol bei 77,6°C (Advances in Chem. Ser. 116, S. 316). Eine einfache destillative Trennung des Systems Cyclohexen/Cyclohexan erfordert einen sehr hohen und unwirtschaftlichen Trennaufwand (J. Gmehling, U. Onken, W. Arlt bzw. B. Kolbe, Vapor-Liquid Equilibrium Data Series, Vol. I, Part. 6a, S. 138 u. Part 6c, S. 166–170).

Über das Siedeverhalten des ternären Systems Cyclohexen/Cyclohexan und Benzol ist nichts bekannt.

Da wegen der Komplexheit des Systems eine destillative Trennung der Komponenten zu aufwendig erschien, wurden in der Patentliteratur zur Isolierung von Cyclohexen aus seinen Gemischen mit Cyclohexan und Benzol Extraktivdestillationen vorgeschlagen. So werden in der JA 49 135 (JP-A 51 127 043) (24.04.75) Dimethylsulfoxid, in der JA 81 418 (JP-A 525 733) (3.7.75) N-Methylpyrrolidon, in der JA 60 192 (JP-A 52 144 549) (26.05.76) Dimethylformamid und in der JA 60 193 (JP-A 52 144 650) Butyrolacton als Flüssigphase mit hoher Selektivität für Cyclohexen bei der Extraktivdestillation beschrieben.

In Analogie zur Aromatengewinnung wäre eine Benzolabtrennung durch Azeotropdestillation oder Extraktion durchführbar (Eisenlohr, Erdöl und Kohle, Erdgas, Petrochemie 1963, 523–533; Ullmanns Enzykl. d. techn. Chem. 1974, 4. Aufl., Bd. 8, S. 392–402; Cinelli et al, Hydrocarbon processing, 1972, S. 141–144; Müller, Chemiker-Zeitung 95, 1971, S. 996–1000). Das nach der Benzolabtrennung resultierende Cyclohexen/Cyclohexan-Gemisch müßte sehr aufwendig durch Rektifikation oder durch Extraktiv-Destillation getrennt werden.

Alle beschriebenen Vorschläge zur Lösung dieses Trennproblems sind unwirtschaftlich und verfahrenstechnisch unbefriedigend.

Der Erfindung lag daher die Aufgabe zugrunde, ein einfaches und wirtschaftliches Verfahren zur Abtrennung oder Anreicherung von Cyclohexen aus seinen Gemischen mit Cyclohexan und Benzol bereitzustellen.

Demgemäß wurde ein Verfahren zur destillativen Aufarbeitung von Cyclohexen, Cyclohexan und Benzol enthaltenden Gemischen gefunden, welches dadurch gekennzeichnet ist, daß man von solchen Gemischen ausgeht, in denen das Molverhältnis $q$ von Benzol zu Cyclohexan 0,1:1 bis 10:1 beträgt, oder daß man Gemische anderer Molverhältnisse $q'$ durch Zugabe von Benzol bzw. Cyclonhexan auf einen Wert des Bereiches $q$ stellt und Benzol und Cyclohexan als Azeotrop durch fraktionierte Destillation vom Cyclohexen abtrennt.

Besondere Ausführungsformen dieses Verfahrens beruhen darauf, das Verhältnis $q$ bereits durch geeignete Wahl der Hydrierungsbedingungen von Benzol oder Behydrierungsbedingungen von Cyclohexan einzustellen.

Das Gemisch wird in der Weise rektifiziert, daß das azeotrope Gemisch aus Benzol und Cyclohexan, das noch geringe Mengen Cyclohexen und gegebenenfalls Verunreinigungen, die bei der Hydrierung bzw. Dehydrierung entstehen, enthalten kann, über Kopf destilliert wird. Im Sumpf verbleibt Cyclohexen entweder in praktisch reiner oder in stark angereicherter Form neben Benzol und/oder Cyclohexan.

Für die Destillation eignen sich Kolonnen beliebiger Bauart, also z.B. Glockenboden-, Siebboden- sowie vor allem Füllkörperkolonnen. Sie werden bei Atmosphären-, Unter- oder Überdruck betrieben. Vorteilhaft wird das Cyclohexen, Cyclohexan und Benzol enthaltende Gemisch im Verlauf der Kolonne im zweiten oder dritten Viertel, insbesondere im dritten Viertel, zugegeben.

Damit das Cyclohexen in reiner bzw. stark angereicherter Form und guten Ausbeuten zu gewinnen ist, sind 30 bis 150, vorzugsweise 40 bis 120 theoretische Trennstufen und Rücklaufverhältnisse von 5 bis 400, vorzugsweise 6 bis 100 zu empfehlen. Geringere Trennstufenzahlen und Rücklaufverhältnisse können verwendet werden; dabei verringert sich allerdings die Cyclohexenausbeute bzw. die Cyclohexenkonzentration im Sumpf.

Im wesentlichen hängen Reinheit bzw. Konzentration des Cyclohexens im Sumpf jedoch vom Molverhältnis $q$ des Benzol/Cyclohexan-Gemisches ab. Dieses Verhältnis $q$ liegt zwischen 0,1:1 und 10:1, bevorzugt 0,2 und 5 und besonders bevorzugt zwischen 0,9 und 1,6.

Entspricht $q$ dem azeotropen Verhältnis von etwa 1,2:1 ($q_{az.}$), so erhält man als Sumpfprodukt Cyclohexen in einer Reinheit von 99%. Der Sumpf besteht bei Werten über 1,2 aus einem Cyclohexen/Benzol-Gemisch, bei Werten unter 1,2 aus einem Cyclohexen/Cyclohexan-Gemisch.

Diese Gemische sind deshalb von Interesse, da bei einigen Folgereaktionen des Cyclohexens Benzol bzw. Cyclohexan als Lösungsmittel dienen kann.

Die zur Herstellung der Cyclohexen-, Cyclohexan-, Benzol-Gemische angewandte partielle Hydrierung von Benzol wird in an sich bekannter Weise vorzugsweise so durchgeführt, daß man Benzol in Gegenwart von Wasser mit Wasserstoff an gelösten oder suspendierten Ruthenium-Katalysatoren, die gegebenenfalls durch ein oder mehrere Übergangsmetalle und/oder seltene Erdmetalle wie z.B. Eisen, Cobalt, Nickel, Cer und besonders bevorzugt Kupfer oder Silber dotiert sein können, bei Temperaturen von 20 bis 300°C, besonders bevorzugt 50 bis 200°C und einem Druck von 0,1 bis 500 bar, besonders bevorzugt 10 bis 200 bar, bei Verweilzeiten von 0,01 bis 5 h, besonders bevorzugt 0,05 bis 2 h, in der Flüssig- oder Gasphase, diskontinuierlich oder kontinuierlich einsetzt. Als Nebenprodukte bei der Benzolhydrierung treten aliphatische Kohlenwasserstoffe, wie z.B. Butane, Pentane, Hexane auf.

Derartige Verfahren sind z.B. in der US-PS 3 912 787, DE-OS 2 221 137 und der DE-OS 2 520 430 beschrieben.

Die partielle Dehydrierung von Cyclohexen kann in an sich bekannter Weise bevorzugt in Gegenwart von Wasserstoff und Edelmetallkatalysatoren der VIII. Nebengruppe, insbesondere Platin oder Palladium oder Oxiden von Metallen der VI. Nebengruppe wie Chrom- oder Molybdänoxiden bei Temperaturen von 200 bis 650°C, bevorzugt 300 bis 600, bei einem Druck von 0,01 bis 10 bar, bevorzugt 0,1 bis 5 bar, in der Flüssig- oder Gasphase, diskontinuierlich oder kontinuierlich durchgeführt werden. Ein geeignetes Verfahren wird z.B. beschreiben in der EP-Patentschrift 23 379.

Durch geeignete Wahl des Katalysators, der Temperatur, des Druckes und insbesondere der Verweilzeit kann die Benzolhydrierung bzw. Cyclohexan-Dehydrierung so gesteuert werden, daß das Benzol/Cyclohexan-Molverhältnis q, darunter das besonders bevorzugte Verhältnis $q_{az.}$, gezielt eingestellt werden kann.

Wird das erfindungsgemäße Benzol/Cyclohexan-Molverhältnis q durch die Benzol-Hydrierung bzw. Cyclohexan-Dehydrierung nicht erreicht, so kann das Verhältnis durch Zugabe von Benzol oder Cyclohexan eingestellt werden.

Das Cylcohexen ist ein wichtiges Zwischenprodukt, das u.a. zur Herstellung von Cyclohexanol durch Wasseranlagerung dienen kann.

Beispiel 1

In einer Füllkörperkolonne mit 83 theoretischen Trennstufen (30 im Verstärkungs- und 53 im Abtriebsteil) wurden stündlich 81 g eines Gemisches aus 20 Mol.-% Cyclohexen, 44 Mol.-% Benzol und 36 Mol.-% Cyclohexan (das entspricht einem Benzol/Cyclohexan-Molverhältnis q von 1,22) bei 1 bar und einem Rückflußverhältnis von 14,5 kontinuierlich destilliert. Bei 77°C erhielt man 65 g/h eines Kopfproduktes aus (laut GC-Analyse) 54,5 Mol.-% Benzol, 45 Mol.-% Cyclohexan und 0,5 Mol.-% Cyclohexen. Dem Sumpf wurden stündlich 16 g eines Gemisches aus 99,1 Mol.-% Cyclohexen, 0,7 Mol.-% Cyclohexan und 0,2 Mol.-% Benzol entnommen. Dies bedeutet, daß 97,5% des im Zulauf enthaltenen Cyclohexens als Sumpfprodukt isoliert wurden.

Beispiel 2

In einer Füllkörperkolonne mit 70 theoretischen Trennstufen (25 im Verstärkungs- und 45 im Abtriebsteil) wurden stündlich 81 g eines Gemisches aus 50 Mol.-% Cyclohexen, 30 Mol.-% Benzol und 20 Mol.-% Cyclohexan (das entspricht einem Benzol/Cyclohexan-Molverhältnis von 1,5) bei 1,5 bar und einem Rücklaufverhältnis von 35 kontinuierlich destilliert. Bei 90°C erhielt man 41,5 g/h eines Kopfproduktes aus (laut GC-Analyse) 57,5 Mol.-% Benzol, 38 Mol.-% Cyclohexan und 4,5 Mol.-% Cyclohexen. Dem Sumpf wurden stündlich 39,5 g eines Gemisches aus 99,0 Mol.-% Cyclohexen, 0,8 Mol.-% Cyclohexan und 0,2 Mol.-% Benzol entnommen. Dies bedeutet, daß 95% des im Zulauf vorhandenen Cyclohexens als Sumpfprodukt isoliert wurden.

Beispiel 3

In einer Füllkörperkolonne mit 83 theoretischen Trennstufen (30 im Verstärkungs- und 53 im Abtriebsteil) wurden stündlich 81 g eines Gemisches aus 4 Mol.-% Cyclohexen, 52 Mol.-% Benzol und 44 Mol.-% Cyclohexan (das entspricht einem Benzol/Cyclohexan-Molverhältnis von 1,18) bei 0,8 bar und einem Rücklaufverhältnis von 14,5 kontinuierlich destilliert. Bei 69–70°C wurden 77,8 g/h eines Kopfproduktes aus (laut GC-Analyse) 54 Mol.-% Benzol, 46 Mol.-% Cyclohexan und 0,1 Mol.-% Cyclohexen entnommen. Dem Sumpf wurden stündlich 3,2 g eines Gemisches aus 99,5 Mol.-% Cyclohexen, 0,3 Mol.-% Cyclohexan und 0,2 Mol.-% Benzol entnommen. Dies bedeutet, daß 97% des im Zulauf vorhandenen Cyclohexens als Sumpfprodukt isoliert wurden.

Beispiel 4

In einer Füllkörperkolonne mit 70 theoretischen Trennstufen (32 im Verstärkungs- und 38 im Abtriebsteil) wurden stündlich 81 g eines Gemisches aus 70 Mol.-% Cyclohexen, 24 Mol.-% Benzol und 6 Mol.-% Cyclohexan (das entspricht einem Benzol/Cyclohexan-Molverhältnis von 4,00) bei 1 bar und einem Rückflaufverhältnis von 40 kontinuierlich destilliert. Bei 78°C erhielt man 24 g/h eines Kopfproduktes aus (laut GC-Analyse) 64 Mol.-% Benzol, 19 Mol.-% Cyclohexan und 17 Mol.-% Cyclohexen. Dem Sumpf wurden stündlich 57 g eines Gemisches aus 92,5 Mol.-% Cyclohexen, 0,5 Mol.-% Cyclohexan und 7.0 Mol.-% Benzol entnommen. Dies bedeutet, daß 92,5% des im Zulauf enthaltenen Cyclohexens als Sumpfprodukt isoliert wurden.

Beispiel 5

In einer Füllkörperkolonne mit 70 theoretischen Trennstufen (40 im Verstärkungs- und 30 im Abtriebsteil) wurden stündlich 82 g eines Gemisches aus 90 Mol.-% Cyclohexen, 2 Mol.-% Benzol und 8 Mol.-% Cyclohexan (das entspricht einem Benzol/Cyclohexan-Molverhältnis von 0,25) bei 0,7 bar und einem Rücklaufverhältnis von 60 kontinuierlich destilliert. Als Kopfprodukt erhielt man 8 g/h eines Gemisches aus (laut GC-Analyse) 20 Mol.-% Benzol, 32,5 Mol.-% Cyclohexan und 47,5 Mol.-% Cyclohexen mit dem Siedepunkt 68–69°C. Dem Sumpf wurden stündlich 74 g eines Gemisches aus 95 Mol.-% Cyclohexen, 5 Mol.-% Cyclohexan und <0,001 Mol.-% Benzol entnommen. Dies bedeutet, daß 94,5% des im Zulauf vorhandenen Cyclohexens als Sumpfprodukt isoliert wurden.

Beispiel 6

In einer Füllkörperkolonne mit 95 theoretischen Trennstufen (67 im Verstärkungs- und 28 im Abtriebsteil) wurden stündlich 82 g eines Gemisches aus 99,0 Mol.-% Cyclohexen, 0,55 Mol.-% Benzol und 0,45 Mol.-% Cyclohexan (das entspricht einem Benzol/Cyclohexan-Molverhältnis von 1,2) bei 1 bar und einem Rücklaufverhältnis von 275 kontinuierlich destilliert. Als Kopfprodukt erhielt man 4 g/h eines Gemisches aus (laut GC-Analyse) 11 Mol.-% Benzol, 6 Mol.-% Cyclohexan und 83 Mol.-% Cyclohexen, mit dem Siedepunkt 82°C. Dem Sumpf wurden stündlich 78 g eines Gemisches aus 99,85 Mol.-% Cyclohexen, 0,15 Mol.-% Cyclohexan und <0,0001 Mol.-% Benzol entnommen. Dies bedeutet, daß 95% des im Zulauf vorhandenen Cyclohexens als Sumpfprodukt isoliert wurden.

Beispiel 7

In einer Füllkörperkolonne mit 90 theoretischen Trennstufen (35 im Verstärkungs- und 55 im Abtriebsteil) wurden stündlich 100 g eines Gemisches aus 17 Mol.-% Cyclohexen, 45,5 Mol.-% Benzol und 37,5 Mol.-% Cyclohexan (Molverhältnis Benzol:Cyclohexan = 1,22) bei 1,0 bar und einem Rücklaufverhältnis von 9 kontinuierlich destilliert. Bei 77°C erhielt man 83 g/h eines Kopfproduktes mit der Zusammensetzung (laut GC-Analyse) 54,5 Mol.-% Benzol, 45,0 Mol.-% Cyclohexan und 0,5 Mol.-% Cyclohexen. Dem Sumpf wurden stündlich 17 g eines Gemisches mit der Zusammensetzung 97,2 Mol.-% Cyclohexen, 2,2 Mol.-% Cyclohexan und 0,6 Mol.-% Benzol entnommen. Dies bedeutet, daß 96,8% des im Zulauf enthaltenen Cyclohexens als Sumpfprodukt isoliert wurden.
Das Ausgangsgemisch wurde wie folgt hergestellt:
Durch einen Rohrreaktor (Länge 50 cm, Durchmesser 0,6 cm) wurden bei 170°C und 80 bar Wasserstoff (10 l Abgas/Stunde) pro Stunde 100 ml Benzol und 50 ml Wasser über 5 g eines Ru/Cu/Co/Ce-Katalysators (0,5 Gew.-% Ru, 0,5 Gew.-% Cu, 0,5 Gew.-% Co und 0,5 Gew.-% Ce) auf $Al_2O_3$ als Träger gepumpt. Nach 100 Stunden Reaktionszeit wurden nach Entfernung des Wassers durch Phasentrennung 10 l des Ausgangsgemisches erhalten.

Beispiel 8

In einer Kolonne mit 90 theoretischen Trennstufen (45 im Verstärkungs- und 45 im Abtriebsteil) wurden stündlich 100 g eines Gemisches aus 16 Mol.-% Cyclohexen, 45,5 Mol.-% Benzol und 38,5 Mol.-% Cyclohexan (Molverhältnis Benzol:Cyclohexan = 1,17) bei 1,5 bar und einem Rücklaufverhältnis von 12,5 kontinuierlich destilliert. Bei 90°C erhielt man 83,2 g/h eines Kopfproduktes mit der Zusammensetzung 54,6 Mol.-% Benzol, 45,4 Mol.-% Cyclohexan und < 0,1 Mol.-% Cyclohexen. Dem Sumpf wurden stündlich 16,8 g eines Gemisches mit der Zusammensetzung 95,4 Mol.-% Cyclohexen, 4,1 Mol.-% Cyclohexan und 0,5 Mol.-% Benzol entnommen. Dies bedeutet, daß 99,9% des im Zulauf vorhandenen Cyclohexens als Sumpfprodukt isoliert wurden.
Das Ausgangsgemisch wurde wie folgt hergestellt:
Durch einen Rohrreaktor (Länge 50 cm, Durchmesser 0,6 cm) wurden bei 140°C und 70 bar Wasserstoff (10 l Abgas/Stunde) pro Stunde 50 ml Benzol und 25 ml Wasser über 5 g eines Ru/Cu-Katalysators (0,5 Gew.-% Ru und 1 Gew.-% Cu) auf $Al_2O_3$ als Träger gepumpt. Nach 20 Stunden Reaktionszeit wurden nach Entfernung des Wassers durch Phasentrennung 1000 ml eines Gemisches erhalten, das zu 24 Gew.-% aus Cyclohexen, 61 Gew.-% Cyclohexan und 15 Gew.-% Benzol bestand. Der Austrag wurde mit 465 ml Benzol versetzt, so daß insgesamt 1465 ml eines Gemisches aus 16 Gew.-% Cyclohexen, 40 Gew.-% Cyclohexan und 44 Gew.-% Benzol erhalten wurde.

Beispiel 9

In einer Füllkörperkolonne mit 99 theoretischen Trennstufen (55 im Verstärkungs- und 35 im Abtriebsteil) wurden stündlich 100 g eines Gemisches aus 12 Mol.-% Cyclohexen, 47,5 Mol.-% Benzol und 40,5 Mol.-% Cyclohexan (Molverhältnis Benzol:Cyclohexan = 1,19) bei 0,8 bar und einem Rücklaufverhältnis von 12 kontinuierlich destilliert. Bei 69,5°C erhielt man 87,8 g/h eines Kopfproduktes mit der Zusammensetzung 54,1 Mol.-% Benzol, 45,6 Mol.-% Cyclohexan und 0,3 Mol.-% Cyclohexen. Dem Sumpf wurden stündlich 12,2 g eines Gemisches mit der Zusammensetzung 96,9 Mol.-% Cyclohexen, 2,8 Mol.-% Cyclohexan und 0,3 Mol.-% Benzol entnommen. Dies bedeutet, daß 98,2% des im Zulauf vorhandenen Cyclohexens als Sumpfprodukt isoliert wurden.
Das Ausgangsgemisch wurde wie folgt hergestellt:
Durch einen Rohrreaktor (Länge 50 cm, Durchmesser 0,6 cm) wurden bei 200°C und 90 bar Wasserstoff (10 l Abgas/Stunde) pro Stunde 50 ml Benzol und 25 ml Wasser über 5 g eines Ru/Cu/Co/Ce-Katalysators (0,5 Gew.-% Ru, 0,5 Gew.-% Cu, 0,5 Gew.-% Co und 0,5 Gew.-% Ce) auf $SiO_2$ als Träger gepumpt. Nach 8 Stunden Reaktionszeit wurden nach Entfernung des Wassers durch Phasentrennung 400 ml eines Gemisches erhalten, das zu 15 Gew.-% aus Cyclohexen, 28 Gew.-% Cyclohexan und 57 Gew.-% Benzol bestand. Der Austrag wurde mit 103 ml Cyclohexan versetzt, so daß insgesamt 503 ml eines Gemisches erhalten wurden, das 12 Gew.-% Cyclohexen, 42 Gew.-% Cyclohexan und 46 Gew.-% Benzol enthielt.

Beispiel 10

In einer Füllkörperkolonne mit 90 theoretischen Trennstufen (34 im Verstärkungs- und 56 im Abtriebsteil) wurden stündlich 100 g eines Gemisches aus 6,5 Mol.-% Cyclohexen, 50,6 Mol.-% Benzol und 42,9 Mol.-% Cyclohexan (Molverhältnis Benzol:Cyclohexan = 1,18) bei 1,0 bar und einem Rücklaufverhältnis von 11,5 kontinuierlich destilliert. Bei 76,5°C erhielt man 93,5 g/h eines Kopfproduktes mit der Zusammensetzung 54,1 Mol.-% Benzol, 45,8 Mol.-% Cyclohexan und 0,1 Mol.-% Cyclohexen. Dem Sumpf wurden stündlich 6,5 g mit der Zusammensetzung 98,9 Mol.-% Cyclohexen, 0,2 Mol.-% Cyclohexan und 0,9 Mol.-% Benzol entnommen. Dies bedeutet, daß 99% des im Zulauf vorhandenen Cyclohexens als Sumpfprodukt isoliert wurden.

Das Ausgangsgemisch wurde wie folgt hergestellt:

Durch ein Quarzrohr (Länge 50 cm, Durchmesser 20 mm) wurden bei 575°C pro Stunde 5 ml Cyclohexan und 13 l Wasserstoff über 20 ml eines Ru/Se-Katalysators (4,0 Gew.-% Ru, 1,0 Gew.-% Se) auf SiO2 als Träger geleitet. Nach 48 Stunden Reaktionszeit wurden 240 ml eines Gemisches erhalten, das zu 7 Gew.-% aus Cyclohexen, 48 Gew.-% Cyclohexan und 45 Gew.-% Benzol bestand. Der Austrag wurde mit 17 ml Benzol versetzt, so daß insgesamt 257 ml eines Gemisches erhalten wurden, das 6,5 Gew.-% Cyclohexen, 44,5 Gew.-% Cyclohexan und 49 Gew.-% Benzol enthielt.

## Patentansprüche

1. Verfahren zur destillativen Aufarbeitung von Cyclohexen, Cyclohexan und Benzol enthaltenden Gemischen, dadurch gekennzeichnet, daß man von solchen Gemischen ausgeht, in denen das Molverhältnis q von Benzol zu Cyclohexan 0,1:1 bis 10:1 beträgt oder daß man Gemische anderer Molverhältnisse q' durch Zugabe von Benzol bzw. Cyclohexan auf einen Wert des Bereiches q stellt und Benzol und Cyclohexan als Azeotrop durch Rektifikation vom Cyclohexen abtrennt.

2. Verfahren nach Anspruch 1, dadurch gekennzeichnet, daß man es auf solche Gemische anwendet, die durch katalytische Hydrierung von Benzol unter solchen Bedingungen hergestellt wurden, daß im Reaktionsgemisch bereits ein Verhältnis q vorliegt.

3. Verfahren nach Anspruch 2, dadurch gekennzeichnet, daß die Hydrierung des Benzols in Gegenwart von Wasser an Ruthenium-Katalysatoren bei Temperaturen von 50°C bis 300°C, einem Druck von 0,1 bis 500 bar, Verweilzeiten von 0,01 bis 5 h in der Flüssig- oder Gasphase, diskontinuierlich oder kontinuierlich vorgenommen wurde.

4. Verfahren nach Anspruch 1, dadurch gekennzeichnet, daß man es auf solche Gemische anwendet, die durch katalytische thermische Dehydrierung von Cyclohexan unter solchen Bedingungen hergestellt wurden, daß im Reaktionsgemisch bereits ein Verhältnis q vorliegt.

5. Verfahren nach Anspruch 4, dadurch gekennzeichnet, daß die Dehydrierung des Cyclohexans in Gegenwart von Wasserstoff an Edelmetall-Katalysatoren der VIII. Nebengruppe oder Oxiden von Metallen der VI. Nebengruppe bei Temperaturen von 200 bis 650°C, und einem Druck von 0,01 bis 10 bar diskontinuierlich oder kontinuierlich vorgenommen wurde.

6. Verfahren nach den Ansprüchen 1 bis 5, dadurch gekennzeichnet, daß in den unmittelbar der Rektifikation zugeführten Gemischen ein Molverhältnis qaz. vorliegt oder eingestellt worden ist, welches der Zusammensetzung des Cyclohexan/Benzol-Azeotropes unter dem angewendeten Destillationsdruck entspricht.

## Claims

1. A process for working up a cyclohexene-, cyclohexane- and benzene-containing mixture by distillation, starting from a mixture in which the molar ratio q of benzene:cyclohexane ranges from 0.1:1 to 10:1 or bringing a mixture of other molar ratio q' into the q range by adding benzene or cyclohexane, and separating benzene and cyclohexane from the cyclohexene as an azeotrope by rectification.

2. A process as claimed in claim 1, which is applied to a mixture prepared by catalytic hydrogenation of benzene under such conditions that a ratio q is already present in the reaction mixture.

3. A process as claimed in claim 1, wherein the hydrogenation of benzene was carried out batchwise or continuously in the presence of water over a ruthenium catalyst at from 50°C to 300°C and from 0.1 to 500 bar in the liquid or gas phase within a residence time of from 0.01 to 5 h.

4. A process as claimed, in claim 1, which is applied to a mixture obtained by catalytic thermal dehydrogenation of cyclohexane under such conditions that a ratio q is already present in the reaction mixture.

5. A process as claimed in claim 4, wherein the dehydrogenation of cyclohexane was carried out batchwise or continuously in the presence of hydrogen over a noble metal catalyst of group VIII of the periodic table of the elements or over an oxide of a metal of group VI at from 200 to 650°C and from 0.01 to 10 bar.

6. A process as claimed in claims 1 to 5, wherein, in the mixture subjected directly to rectification, there is present or has been set a molar ratio qaz. which corresponds to the composition of the cyclohexane/benzene azeotrope under the distillation pressure employed.

## Revendications

1. Procédé de traitement par distillation de mélanges contenant du cyclohexène, cyclohexane et benzène, caractérisé par le fait qu'on part de tels mélanges dans lesquels le rapport molaire q du benzène au cyclohexane est de 0,1/1 à 10/1 ou qu'on règle à une valeur du domaine q, par addition de benzène ou cyclohexane, des mélanges d'autres rapports molaires q' et on sépare du cyclohexène, par rectification, du benzène et cyclohexane en tant qu'azéotrope.

2. Procédé selon la revendication 1, caractérisé par le fait qu'il est appliqué à des mélanges qui ont été préparés par hydrogénation catalytique de benzène dans des conditions telles que, dans le mélange de réaction, il existe déjà un rapport q.

3. Procédé selon la revendication 2, caractérisé par le fait que l'hydrogénation du benzène a été effectuée de manière discontinue ou continue, en phase liquide ou gazeuse, en présence d'eau, sur des catalyseurs au ruthénium, à des températures de 50°C à 300°C, à une pression de 0,1 à 500 bar, et des temps de séjour de 0,01 à 5 heures.

4. Procédé selon la revendication 1, caractérisé par le fait qu'il est appliqué à des mélanges qui ont été préparés par deshydrogénation thermique catalytique de cyclohexane dans des conditions telles que, dans le mélange de réaction, existe déjà un rapport q.

5. Procédé selon la revendication 4, caractérisé par le fait que la deshydrogénation du cyclohexane a été effectuée à des températures de 200 à 650°C et une pression de 0,01 à 10 bar, de manière discontinue ou continue, en présence d'hydrogène, sur des catalyseurs sur métal noble du groupe auxiliaire VIII ou oxydes de métaux du groupe auxiliaire VI.

6. Procédé selon les revendications 1 à 5, caractérisé par le fait que, dans les mélanges amenés directement à la rectification, il existe un rapport molaire $q_{az}$ ou ce rapport a été réglé à cette valeur, qui correspond à la composition de l'azéotrope cyclohexane/benzène, sous la pression de distillation appliquée.